# EUROPEAN PATENT APPLICATION

(11) **EP 4 737 474 A1**
(43) Date of publication of application: **06.05.2026**
(21) Application number: 24218621.1
(22) Date of filing: 10.12.2024
(51) Int. Cl.: C07K 14/00, A61K 38/16, A61P 25/28

(54) **SYNTHETIC PEPTIDE, PHARMACEUTICAL COMPOSITION COMPRISING SYNTHETIC PEPTIDE, AND USE OF SYNTHETIC PEPTIDE FOR TREATING AMYLOIDOSIS**

(30) Priority: 04.11.2024 US 202418936483
(71) Applicant: China Medical University Hospital, Taichung City 404327 (TW)
(72) Inventor: Cho, Der-Yang, 404327 Taichung City (TW); Chiu, Shao-Chih, 404327 Taichung City (TW); Huang, Shi-Wei, 404327 Taichung City (TW); Pan, Chih-Ming, 404327 Taichung City (TW); Chen, Mei-Chih, 404327 Taichung City (TW); Lin, Yu-Chuan, 404327 Taichung City (TW); Chen, Yeh, 403521 Taichung City (TW); Wu, Chung-Chun, 404327 Taichung City (TW)
(74) Representative: Scholz, Volker

(57) **Abstract**

The present disclosure provides a synthetic peptide, a pharmaceutical composition including the synthetic peptide, and a use of the synthetic peptide for treating amyloidosis. The synthetic peptide of the present disclosure achieves the effect of treating amyloidosis through various efficacy experiments.

## Description

### BACKGROUND OF THE INVENTION

### 1. Field of the Invention

The present invention relates to a synthetic peptide, a pharmaceutical composition comprising the synthetic peptide, and a use of the synthetic peptide for treating amyloidosis.

### 2. The Prior Art

Amyloid is an insoluble fibrous protein. Abnormal accumulation in organs can cause amyloidosis. In many neurological diseases, such as Alzheimer's disease (AD) and Parkinson's disease (PD), large amounts of amyloid protein accumulation can be observed in the nervous system. Many scholars believe it may cause degeneration or dysfunction of the brain or other organs.

Alzheimer's disease (AD) is characterized by the accumulation of amyloid β (Aβ) in the brain and is the leading cause of dementia worldwide, affecting an increasing number of the elderly population. According to a report by the Alzheimer's Disease International, more than 50 million people worldwide were suffering from dementia in 2020, and this number will double almost every 20 years, reaching 82 million people in 2030. In 2050, it will reach 152 million people. Unfortunately, there is currently no cure for most types of dementia.

If there is one valuable lesson learned from the numerous clinical trial failures of new Alzheimer's drugs, it is that when amyloidβ(Aβ) deposits and tangles have not yet caused irreversible damage to the brain, the disease should be immediately treated with early intervention.

In view of the fact that current drugs for treating amyloidosis still have shortcomings of side effects, chemical synthesis and ineffective effects, in order to solve the above-mentioned problems, those skilled in the art urgently need to develop a novel and more effective medicament for treating amyloidosis for the benefit of a large group of people in need thereof.

### SUMMARY OF THE INVENTION

A primary objective of the present invention is to provide a synthetic peptide, comprising an amino acid sequence of SEQ ID NO:1.

According to an embodiment of the present invention, the synthetic peptide targets amyloid β (Aβ).

According to an embodiment of the present invention, the synthetic peptide prevents formation of amyloid β aggregation.

According to an embodiment of the present invention, the synthetic peptide prevents pan-amyloid β-sheet aggregation.

According to an embodiment of the present invention, the pan-amyloid is amyloid β (Aβ), Tau protein, α-synuclein (α-Syn), Islet amyloid polypeptide (IAPP), or transthyretin (TTR).

According to an embodiment of the present invention, the Tau protein is recombinant Tau P301L protein.

According to an embodiment of the present invention, the TTR is recombinant TTR protein (V122I).

According to an embodiment of the present invention, the TTR is recombinant TTR protein (V30I).

According to an embodiment of the present invention, the synthetic peptide maintains cell viability of 1-methyl-4-phenylpyridin-1-ium (MPP)⁺-treated SH-SY5Y-derived dopaminergic-like neurons.

According to an embodiment of the present invention, the synthetic peptide prevents formation of amyloid aggregates in MPP⁺-treated SH-SY5Y-derived dopaminergic-like neurons.

According to an embodiment of the present invention, the synthetic peptide prevents formation of amyloid aggregates in Aβ-treated SH-SY5Y-derived neuron-like cells.

Another objective of the present invention is to provide a pharmaceutical composition, comprising the above mentioned synthetic peptide and a pharmaceutically acceptable carrier.

Another objective of the present invention is to provide a method for treating amyloidosis, comprising administering to a subject in need thereof the above mentioned pharmaceutical composition.

According to an embodiment of the present invention, the amyloidosis is Parkinson's disease (PD) or Alzheimer's disease (AD).

In summary, the present invention achieves the effect of treating amyloidosis (such as Parkinson's disease and Alzheimer's disease) through the results illustrated in the following examples.

The embodiments of the present invention would be further described below. The following examples are used to illustrate the present invention and are not intended to limit the scope of the present invention. Anyone skilled in the art can make some changes and modifications without departing from the spirit and scope of the present invention. Therefore, the scope of the present invention shall be defined by the appended claims.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.
FIG. 1 shows affinity of PAA peptide to Tau fibrils.
FIG. 2 shows that PAA peptide can prevent pan-amyloid β-sheet aggregation (Aβ).
FIG. 3 shows that PAA peptide can prevent pan-amyloid β-sheet aggregation (Tau).
FIG. 4 shows that PAA peptide can prevent Tau protein (P301L) aggregation.
FIG. 5 shows that PAA peptide can prevent pan-amyloid β-sheet aggregation (α-synuclein (α-Syn)).
FIG. 6 shows that PAA peptide can prevent pan-amyloid β-sheet aggregation (Islet amyloid polypeptide (IAPP)).
FIG. 7 shows that PAA peptide can prevent pan-amyloid β-sheet aggregation (TTR V122I).
FIG. 8 shows that PAA peptide can prevent pan-amyloid β-sheet aggregation (TTR V30I).
FIG. 9 shows PAA peptide uptake by 1-methyl-4-phenylpyridin-1-ium (MPP)⁺-treated SH-SY5Y-derived dopaminergic-like neurons, in which FITC represents fluorescein isothiocyanate.
FIG. 10 shows PAA peptide uptake by Aβ-treated SH-SY5Y-derived neuron-like cells.
FIG. 11 shows that PAA peptide maintains cell viability of MPP⁺-treated SH-SY5Y-derived dopaminergic-like neurons.
FIG. 12 shows that PAA peptide maintains cell viability of Aβ-treated SH-SY5Y-derived neuron-like cells.
FIG. 13 shows that PAA peptide prevents the formation of amyloid aggregates in MPP⁺-treated SH-SY5Y-derived dopaminergic-like neurons.
FIG. 14 shows that PAA peptide prevents the formation of amyloid aggregates in Aβ-treated SH-SY5Y-derived neuron-like cells.

### DETAILED DESCRIPTION OF THE PREFERRED EMBODIMENT

In the following detailed description of the embodiments of the present invention, reference is made to the accompanying drawings, which are shown to illustrate the specific embodiments in which the present disclosure may be practiced. These embodiments are provided to enable those skilled in the art to practice the present disclosure. It is understood that other embodiments may be used and that changes can be made to the embodiments without departing from the scope of the present invention. The following description is therefore not to be considered as limiting the scope of the present invention.

### Definition

As used herein, the data provided represent experimental values that can vary within a range of ±20%, preferably within ±10%, and most preferably within ±5%.

Unless otherwise stated in the context, "a", "the" and similar terms used in the specification (especially in the following claims) should be understood as including singular and plural forms.

As used herein, the term "treating" or "treatment" refers to alleviating, reducing, ameliorating, relieving or controlling one or more clinical signs of a disease or disorder, and lowering, stopping, or reversing the progression of severity regarding the condition or symptom being treated.

According to the present invention, the pharmaceutical composition can be manufactured to a dosage form suitable for parenteral or oral administration, using techniques well known to those skilled in the art, including, but not limited to, injection (e.g., sterile aqueous solution or dispersion), sterile powder, tablet, troche, lozenge, pill, capsule, dispersible powder or granule, solution, suspension, emulsion, syrup, elixir, slurry, and the like.

The pharmaceutical composition according to the present invention may be administered by a parenteral route selected from the group consisting of intraperitoneal injection, subcutaneous injection, intraepidermal injection, intradermal injection, intramuscular injection, intravenous injection, and intralesional injection.

According to the present invention, the pharmaceutical composition may further comprise a pharmaceutically acceptable carrier which is widely used in pharmaceutically manufacturing techniques. For example, the pharmaceutically acceptable carrier can comprise one or more reagents selected from the group consisting of solvent, emulsifier, suspending agent, decomposer, binding agent, excipient, stabilizing agent, chelating agent, diluent, gelling agent, preservative, lubricant, absorption delaying agent, liposome, and the like. The selection and quantity of these reagents fall within the scope of the professional literacy and routine techniques of those skilled in the art.

According to the present invention, the pharmaceutically acceptable carrier comprises a solvent selected from the group consisting of water, normal saline, phosphate buffered saline (PBS), sugar-containing solution, aqueous solution containing alcohol, and combinations thereof.

The present invention is further illustrated by the following examples. These examples are provided for illustration only and are not intended to limit the scope of the present invention. The scope of the present invention is shown in the appended claims.

In this example, the synthetic peptide of the present invention (hereinafter referred to as PAA peptide) may comprise an amino acid sequence of SEQ ID NO:1.

In this example, PAA peptide targets amyloid β for treating Parkinson's disease (PD) and Alzheimer's disease (AD).

### Example 1

### Preparation of synthetic peptide of present invention

In this example, the synthetic peptide (hereinafter referred to as PAA peptide) is prepared by the well-known solid phase peptide synthesis technology.

### Example 2

### Affinity of PAA peptide of present invention to Tau fibrils

In this example, the experimental procedure of the affinity of PAA peptide to Tau fibrils is as follows. Surface plasmon resonance (SPR) analysis for binding affinity of PAA peptide: The CM5 or NTA chip, research grade would be performed for SPR analysis by BIAcore T200 (Biacore-GE Healthcare, Piscataway, NJ). Briefly, dilute protein (Tau recombinant protein) sample in the 10 mM buffer solutions (pH 4.0, 5.5 or 6.0) at the concentration range of 20 µg/mL to give maximum surface retention for immobilization on the chip, following the SURFACE PREPATRATION process and choosing the condition of higher surface concentration of ligands (PAA peptide: 50, 25, 12.5, 6.25, 3.125 and 1.5625 nM) on the chip. Then the regeneration scouting and surface performance test, following REGENERATION SCOUTING and SURPACE PERFORMANCE TEST and then select REGENERATION METHOD to run the experiment. And then select BINDING ANALYSIS and DIRECT BINDING to investigate protein binding. The KINETIC ANALYSIS will be selected and choose MASS TRANSFER to run kinetic assay accompany with binding experiment. Data analysis and kinetic constants determine.

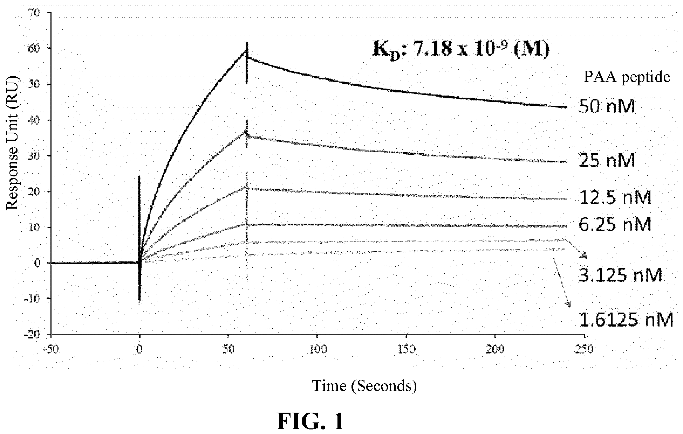

FIG. 1 shows affinity of PAA peptide to Tau fibrils. As shown in FIG. 1, SPR affinity analysis results show that the affinity of PAA peptide for Tau protein is approximately KD = 7.18 × 10 nM.

### Example 3

### Cell-free model for testing whether PAA peptide prevents amyloid β aggregation

In this example, the experimental procedure of cell-free model for testing whether PAA peptide prevents amyloid β aggregation is as follows. After coating with heparin (50 IU/ml, 50 ml) at 37°C for 30 min, recombinant amyloid proteins (50 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader.

### Example 4

### PAA peptide can prevent pan-amyloid β-sheet aggregation (amyloid β (Aβ))

The procedure of this example is as follows. After coating with heparin (50 TU/ml, 50 µl) at 37°C for 30 min, recombinant Aβ peptide (1-42) (50 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. *p < 0.05, **p < 0.01, ***p < 0.001.

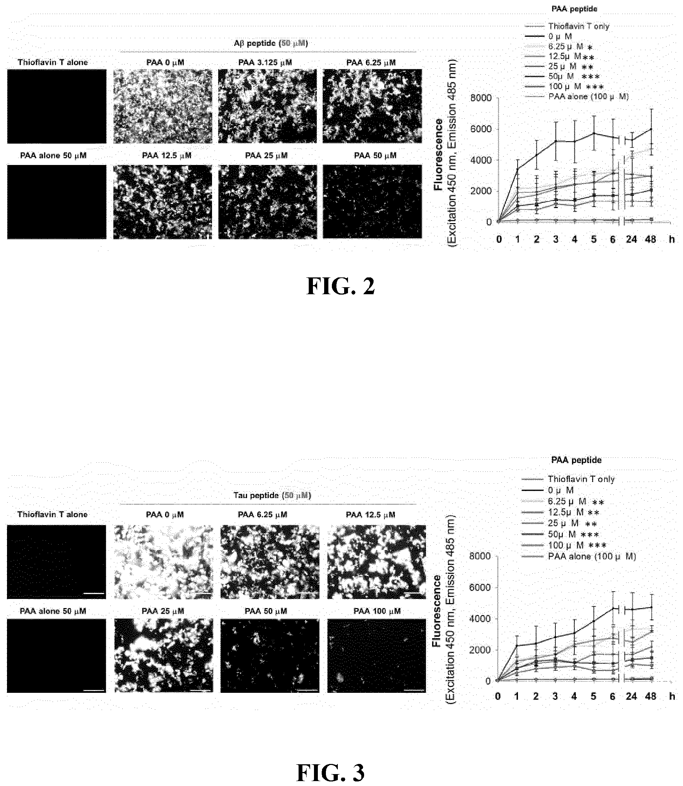

FIG. 2 shows that PAA peptide can prevent pan-amyloid β-sheet aggregation (Aβ). As shown in FIG. 2, PAA peptide can prevent pan-amyloid β-sheet aggregation (Aβ), and the 24-hour fluorescence imaging results (left) and fluorescence quantification results at each time point (right) show a dose-dependent inhibitory effect on Aβ aggregation.

### Example 5

### PAA peptide can prevent pan-amyloid β-sheet aggregation (Tau)

The procedure of this example is as follows. After coating with heparin (50 TU/ml, 50 µl) at 37°C for 30 min, recombinant Tau protein (50 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. **p < 0.01, ***p < 0.001.

FIG. 3 shows that PAA peptide can prevent pan-amyloid β-sheet aggregation (Tau). As shown in FIG. 3, PAA peptide can prevent amyloid Tau folding and aggregation (Tau), and the 24-hour fluorescence imaging results (left) and fluorescence quantification results at each time point (right) show a dose-dependent inhibitory effect on Tau aggregation.

### Example 6

### PAA peptide can prevent Tau protein (P301L) aggregation

The procedure of this example is as follows. After coating with heparin (50 TU/ml, 50 µl) at 37°C for 30 min, recombinant Tau P301L protein (50 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. *p < 0.05, **p < 0.01, ***p < 0.001.

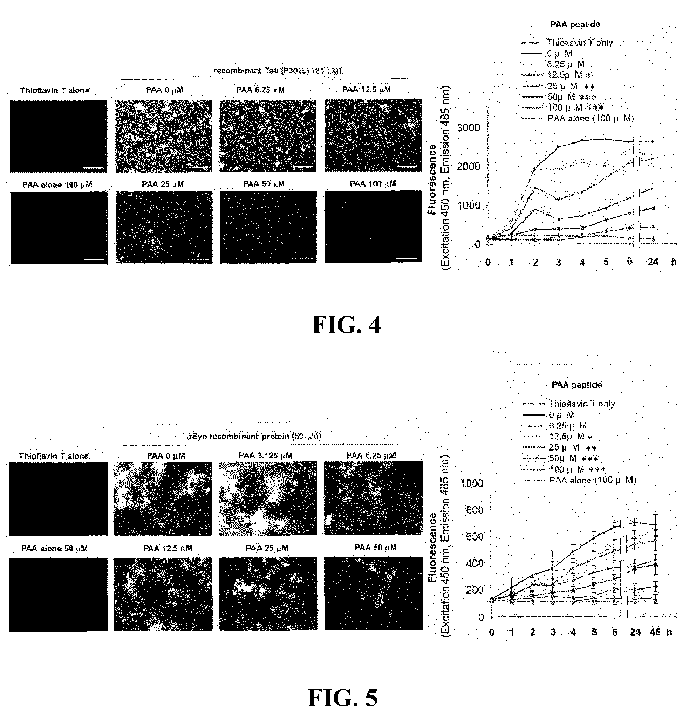

FIG. 4 shows that PAA peptide can prevent Tau protein (P301L) aggregation. As shown in FIG. 4, PAA peptide can prevent amyloid Tau protein (P301L) folding and aggregation, and the 24-hour fluorescence imaging results (left) and fluorescence quantification results at each time point (right) show a dose-dependent inhibitory effect on Tau (P301L) aggregation.

### Example 7

### PAA peptide can prevent pan-amyloid β-sheet aggregation (α-synuclein (α-Syn))

The procedure of this example is as follows. After coating with heparin (50 TU/ml, 50 µl) at 37°C for 30 min, recombinant α-synuclein (α-Syn) preformed fibril (10 µM) and monomer (50 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. *p < 0.05, **p < 0.01, ***p < 0.001.

FIG. 5 shows that PAA peptide can prevent pan-amyloid β-sheet aggregation (α-synuclein (α-Syn)). As shown in FIG. 5, PAA peptide can prevent α-Syn folding and aggregation, and the 24-hour fluorescence imaging results (left) and fluorescence quantification results at each time point (right) show a dose-dependent inhibitory effect on α-Syn aggregation.

### Example 8

### PAA peptide can prevent pan-amyloid β-sheet aggregation (Islet amyloid polypeptide (IAPP))

The procedure of this example is as follows. After coating with heparin (50 TU/ml, 50 µl) at 37°C for 30 min, recombinant Islet amyloid polypeptide (IAPP) peptide (1-37) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. *p < 0.05, **p < 0.01, ***p < 0.001.

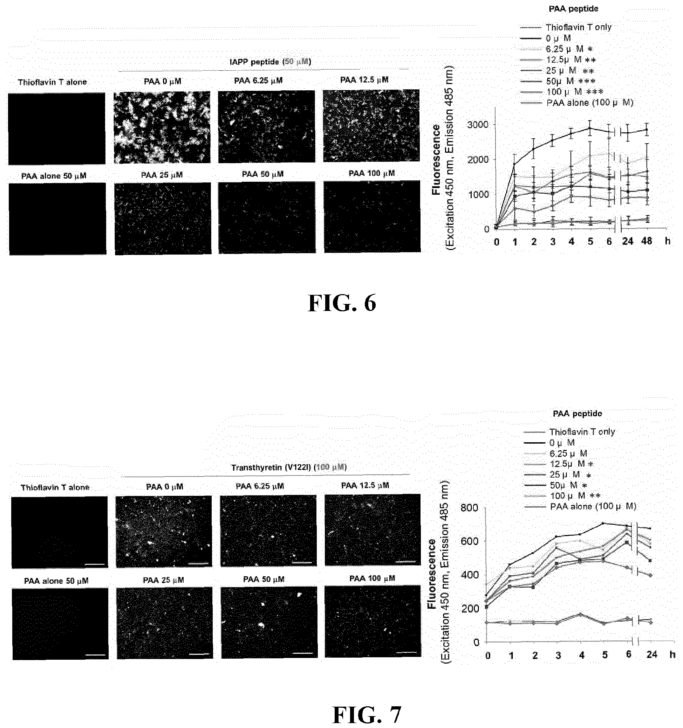

FIG. 6 shows that PAA peptide can prevent pan-amyloid β-sheet aggregation (Islet amyloid polypeptide (IAPP)). As shown in FIG. 6, PAA peptide can prevent IAPP folding and aggregation, and the 24-hour fluorescence imaging results (left) and fluorescence quantification results at each time point (right) show a dose-dependent inhibitory effect on IAPP aggregation.

### Example 9

### PAA peptide can prevent pan-amyloid β-sheet aggregation (TTR V122I)

The procedure of this example is as follows. After coating with heparin (50 TU/ml, 50 µl) at 37°C for 30 min, recombinant Transthyretin (TTR) protein (V122I) (100 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. *p < 0.05, **p < 0.01.

FIG. 7 shows that PAA peptide can prevent pan-amyloid β-sheet aggregation (TTR V122I). As shown in FIG. 7, PAA peptide can prevent TTR V122I folding and aggregation, and the 24-hour fluorescence imaging results (left) and fluorescence quantification results at each time point (right) show a dose-dependent inhibitory effect on TTR V122I aggregation.

### Example 10

### PAA peptide can prevent pan-amyloid β-sheet aggregation (TTR V30I)

The procedure of this example is as follows. After coating with heparin (50 TU/ml, 50 µl) at 37°C for 30 min, recombinant TTR protein (V30I) (100 µM) was added into each well of 96-well plate. Subsequently, these wells were incubated with 0, 3.125, 6.25, 12.5, 25 and 50 µM PAA peptide and supplemented with 25 nM Thioflavin. At the indicated hours post incubation, the fluorescent signals were measured by ELISA reader. Scale bar = 50 µm. Data were presented as mean ± SEM and analyzed using one-way ANOVA by comparing with 0 µM control. Statistical significance was set at p value < 0.05. *p < 0.05, **p < 0.01.

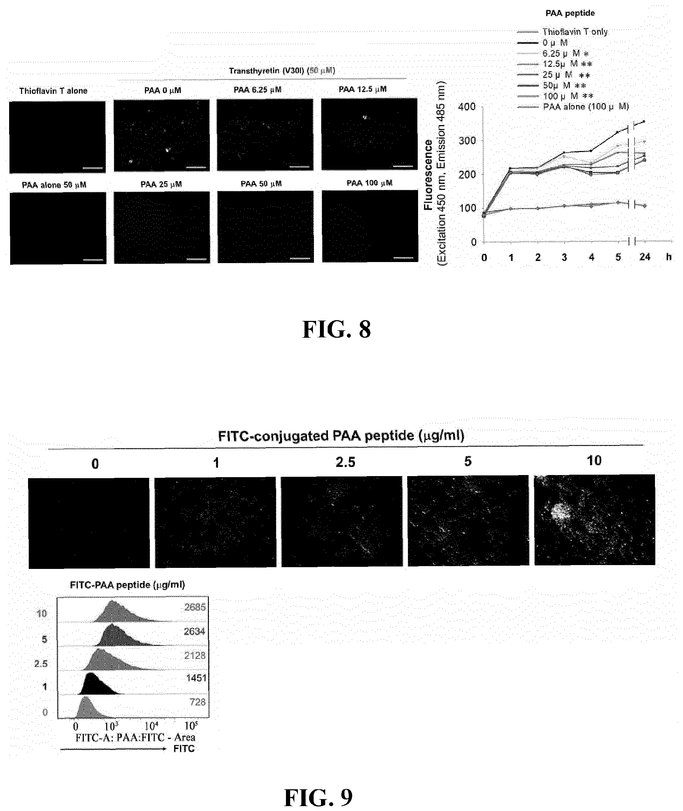

FIG. 8 shows that PAA peptide can prevent pan-amyloid β-sheet aggregation (TTR V30I). As shown in FIG. 8, PAA peptide can prevent TTR V30I folding and aggregation, and the 24-hour fluorescence imaging results (left) and fluorescence quantification results at each time point (right) show a dose-dependent inhibitory effect on TTR V30I aggregation.

### Example 11 PAA peptide uptake by MPP⁺-treated SH-SY5Y-derived dopaminergic-like neurons

The procedure of this example is as follows. 1-methyl-4-phenylpyridin-1-ium (MPP)⁺-treated SH-SY5Y-derived dopaminergic-like neurons were treated with FITC-conjugated PAA peptide (0, 1, 2.5, 5, 10 µg/ml) for 4 h, then the fluorescent signals were detected by microscope (upper panel) and flow cytometry (lower panel).

FIG. 9 shows PAA peptide uptake by 1-methyl-4-phenylpyridin-1-ium (MPP)⁺-treated SH-SY5Y-derived dopaminergic-like neurons, in which FITC represents fluorescein isothiocyanate. As shown in FIG. 9, as the dose of PAA peptide increases, the uptake of PAA peptide by MPP⁺-treated SH-SY5Y-derived dopaminergic-like neuron cells increases from fluorescence imaging (top) and flow cytometry analysis (bottom).

### Example 12

### PAA peptide uptake by Aβ-treated SH-SYSY-derived neuron-like cells

The procedure of this example is as follows. Aβ-treated SH-SY5Y-derived neuron-like cells were treated with FITC-conjugated PAA peptide (0, 1, 2.5, 5, 10 µg/ml) for 4 h, then the fluorescent signals were detected by microscope (upper panel) and flow cytometry (lower panel).

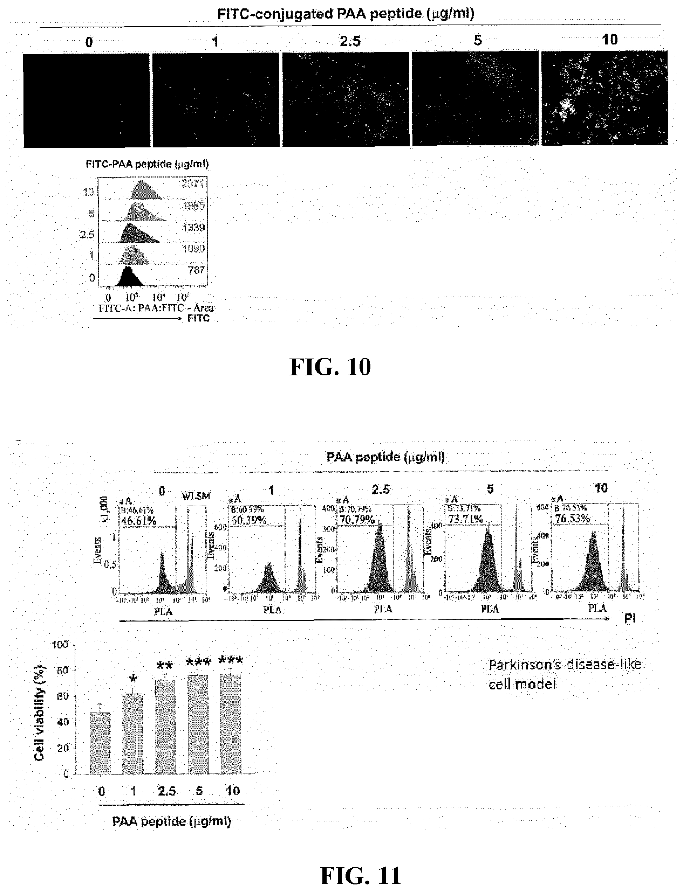

FIG. 10 shows PAA peptide uptake by Aβ-treated SH-SY5Y-derived neuron-like cells. As shown in FIG. 10, as the dose of PAA peptide increases, the uptake of PAA peptide by Aβ-treated SH-SY5Y-derived dopaminergic-like neuron cells increases from fluorescence imaging (top) and flow cytometry analysis (bottom).

### Example 13

### PAA peptide maintains cell viability of MPP⁺-treated SH-SY5Y-derived dopaminergic-like neurons

The procedure of this example is as follows. MPP⁺-treated SH-SY5Y-derived dopaminergic-like neurons were treated with PAA peptide (0, 1, 2.5, 5, 10 µg/ml) for 96 h, then their cell viability was determined by propidium iodide (PI) staining using flow cytometry analysis.

FIG. 11 shows that PAA peptide maintains cell viability of MPP⁺-treated SH-SY5Y-derived dopaminergic-like neurons. As shown in FIG. 11, flow cytometry analysis results show that treatment with PAA peptide can significantly reduce cell death of MPP⁺-treated SH-SY5Y-derived dopaminergic-like neurons.

### Example 14

### PAA peptide maintains cell viability of Aβ-treated SH-SY5Y-derived neuron-like cells

The procedure of this example is as follows. Aβ-treated SH-SY5Y-derived neuron-like cells were treated with PAA peptide (0, 1, 2.5, 5, 10 µg/ml) for 96 h, then their cell viability was determined by PI staining using flow cytometry analysis.

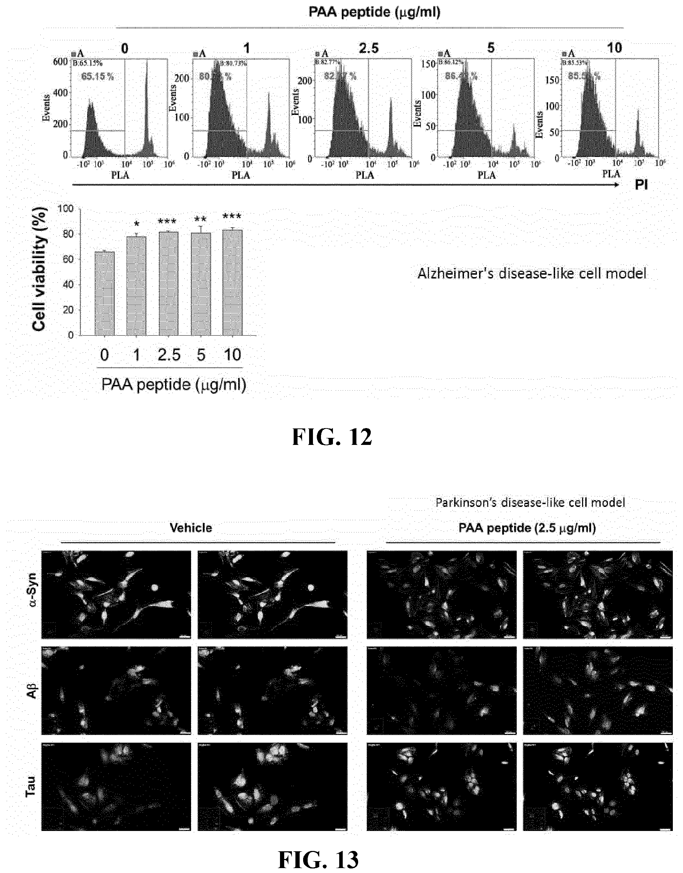

FIG. 12 shows that PAA peptide maintains cell viability of Aβ-treated SH-SY5Y-derived neuron-like cells. As shown in FIG. 12, flow cytometry analysis results show that treatment with PAA peptide can significantly reduce cell death of Aβ-treated SH-SY5Y-derived dopaminergic-like neurons.

### Example 15

### PAA peptide prevents formation of amyloid aggregates in MPP⁺-treated SH-SY5Y-derived dopaminergic-like neurons

The procedure of this example is as follows. After treating MPP⁺-treated SH-SY5Y-derived dopaminergic-like neurons with PAA peptide (2.5 µg/ml) for 96 hours, immunofluorescence staining was used to detect the expression of Aβ, Tau and α-Syn. The aggregation was then observed using confocal microscopy.

FIG. 13 shows that PAA peptide prevents the formation of amyloid aggregates in MPP⁺-treated SH-SY5Y-derived dopaminergic-like neurons. As shown in FIG. 13, fluorescence microscopy imaging results show that treatment with PAA peptide can significantly reduce the aggregation of Aβ, Tau and α-Syn in MPP⁺-treated SH-SY5Y-derived dopaminergic-like neuronal cells.

### Example 16

### PAA peptide prevents formation of amyloid aggregates in Aβ-treated SH-SY5Y-derived neuron-like cells

The procedure of this example is as follows. After treating Aβ-treated SH-SY5Y-derived neuron-like cells with PAA peptide (2.5 µg/ml) for 96 hours, immunofluorescence staining was used to detect the expression of Aβ, Tau and α-Syn. The aggregation was then observed using confocal microscopy.

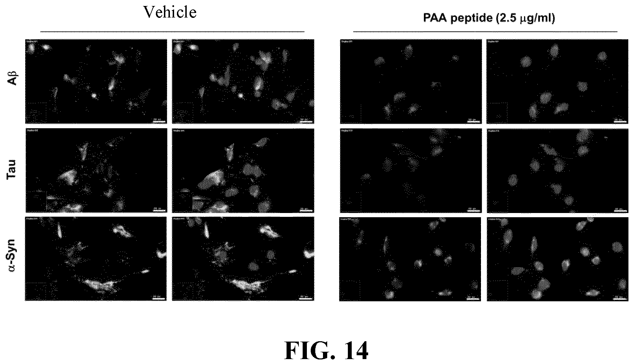

FIG. 14 shows that PAA peptide prevents the formation of amyloid aggregates in Aβ-treated SH-SY5Y-derived neuron-like cells. As shown in FIG. 14, fluorescence microscopy imaging results show that treatment with PAA peptide can significantly reduce the aggregation of Aβ, Tau and α-Syn in Aβ-treated SH-SY5Y-derived neuron-like cells.

In summary, the present invention achieves the effect of treating amyloidosis (such as Parkinson's disease and Alzheimer's disease) through the results illustrated in the above examples.

Although the present invention has been described with reference to the preferred embodiments, it will be apparent to those skilled in the art that a variety of modifications and changes in form and detail may be made without departing from the scope of the present invention defined by the appended claims.

## Claims

1. A synthetic peptide, comprising an amino acid sequence of SEQ ID NO:1.

2. The synthetic peptide according to claim 1, which targets amyloid β (Aβ).

3. The synthetic peptide according to claim 2, which prevents formation of amyloid β aggregation.

4. The synthetic peptide according to claim 3, which prevents pan-amyloid β-sheet aggregation.

5. The synthetic peptide according to claim 4, wherein the pan-amyloid is amyloid β (Aβ), Tau protein, α-synuclein (α-Syn), Islet amyloid polypeptide (IAPP), or transthyretin (TTR).

6. The synthetic peptide according to claim 5, wherein the Tau protein is recombinant Tau P301L protein.

7. The synthetic peptide according to claim 5, wherein the TTR is recombinant TTR protein (V122I).

8. The synthetic peptide according to claim 5, wherein the TTR is recombinant TTR protein (V30I).

9. The synthetic peptide according to claim 1, which maintains cell viability of 1-methyl-4-phenylpyridin-1-ium (MPP)⁺-treated SH-SY5Y-derived dopaminergic-like neurons.

10. The synthetic peptide according to claim 1, which prevents formation of amyloid aggregates in MPP⁺-treated SH-SY5Y-derived dopaminergic-like neurons.

11. The synthetic peptide according to claim 1, which prevents formation of amyloid aggregates in Aβ-treated SH-SY5Y-derived neuron-like cells.

12. A pharmaceutical composition, comprising the synthetic peptide according to claim 1 and a pharmaceutically acceptable carrier.

13. The pharmaceutical composition according to claim 12 for use in the treatment of amyloidosis.

14. The pharmaceutical composition for use according to claim 13, wherein the amyloidosis is Parkinson's disease (PD) or Alzheimer's disease (AD).

## Amended claims

### Amended claims in accordance with Rule 137(2) EPC.

1. A synthetic peptide for treating amyloidosis, comprising an amino acid sequence of SEQ ID NO:1, which prevents pan-amyloid β-sheet aggregation.

2. The synthetic peptide according to claim 1, which targets amyloid β (Aβ).

3. The synthetic peptide according to claim 1, wherein the pan-amyloid is amyloid β (Aβ), Tau protein, α-synuclein (α-Syn), Islet amyloid polypeptide (IAPP), or transthyretin (TTR).

4. The synthetic peptide according to claim 3, wherein the Tau protein is recombinant Tau P301L protein.

5. The synthetic peptide according to claim 3, wherein the TTR is recombinant TTR protein (V1221).

6. The synthetic peptide according to claim 3, wherein the TTR is recombinant TTR protein (V30I).

7. The synthetic peptide according to claim 1, which maintains cell viability of 1-methyl-4-phenylpyridin-1-ium (MPP)⁺-treated SH-SY5Y-derived dopaminergic-like neurons.

8. The synthetic peptide according to claim 1, which prevents formation of amyloid aggregates in MPP⁺-treated SH-SY5Y-derived dopaminergic-like neurons.

9. The synthetic peptide according to claim 1, which prevents formation of amyloid aggregates in Aβ-treated SH-SY5Y-derived neuron-like cells.

10. A pharmaceutical composition, comprising the synthetic peptide according to claim 1 and a pharmaceutically acceptable carrier.

11. The pharmaceutical composition according to claim 10 for use in the treatment of amyloidosis.

12. The pharmaceutical composition for use according to claim 11, wherein the amyloidosis is Parkinson's disease (PD) or Alzheimer's disease (AD). 1
